# EUROPÄISCHE PATENTSCHRIFT

(11) **EP 0 726 732 B1**
(45) Veröffentlichungstag und Bekanntmachung des Hinweises auf die Patenterteilung: **15.07.1998**
(21) Anmeldenummer: 94930937.1
(22) Anmeldetag: 28.10.1994
(51) Int. Cl.: A61B 17/28

(54) **INSTRUMENT ZUR APPLIKATION VON PROTHESEN IM KÖRPERINNEREN**
INSTRUMENT FOR PLACING PROSTHESES INSIDE THE BODY
INSTRUMENT POUR LA MISE EN PLACE DE PROTHESES A L'INTERIEUR DU CORPS

(30) Priorität: 02.11.1993 DE 4337370; 26.05.1994 DE 4418449
(43) Veröffentlichungstag der Anmeldung: 21.08.1996
(73) Patentinhaber: Karl Storz GmbH & Co., 78532 Tuttlingen (DE)
(72) Erfinder: FREITAG, Lutz, D-45276 Essen (DE)
(74) Vertreter: Münich, Wilhelm, Dr.
(86) Internationale Anmeldenummer: DE9401269
(87) Internationale Veröffentlichungsnummer: WO9512356

(56) Entgegenhaltungen:
- DE-A- 3 812 165
- DE-U- 9 109 113
- GB-A- 1 205 743

## Beschreibung

### Technisches Gebiet

Die Erfindung bezieht sich auf ein Instrument zur Applikation von Prothesen im Körperinneren und insbesondere von Tracheo-Bronchial-Prothesen gemäß dem Oberbegriff des Patentanspruchs 1.

### Stand der Technik

Zum Einsetzen beispielsweise von Tracheo-Bronchial-Prothesen werden bislang zwei Instrumente benötigt: Zum einen eine Faßzange, mit der die Prothese gegriffen und in den Tracheo-Bronchial-Raum eingebracht wird; zum anderen ein Hilfsinstrument, mit dem die Prothese während der Entnahme der Faßzange in der gewünschten Lage festgehalten wird.

Eine Faßzange, die insbesondere als Einführungszange für einen Trachealtubus konzipiert ist, ist auf der DE-A-38 12 164 bekannt. Von dieser bekannten Faßzange ist im übrigen bei der Formulierung des Oberbegriffs des Patentanspruchs 1 ausgegangen worden.

Da der verfügbare Querschnitt (Lumen) beim Arbeiten im Körperinneren begrenzt ist, bereitet die gleichzeitige Handhabung einer Faßzange und eines Hilfsinstruments häufig Schwierigkeiten.

### Darstellung der Erfindung

Der Erfindung liegt die Aufgabe zugrunde, ein Instrument zur Applikation von Prothesen im Körperinneren und insbesondere von Tracheo-Bronchial-Prothesen gemäß dem Oberbegriff des Patentanspruchs 1 derart weiterzubilden, daß das komplikationslose Einsetzen einer Prothese möglich ist, ohne daß ein zusätzliches Hilfsinstrument benötigt würde.

Eine erfindungsgemäße Lösung dieser Aufgabe ist im Patentanspruch 1 angegeben. Weiterbildungen der Erfindung sind Gegenstand der Unteransprüche.

Die Erfindung geht von dem Grundgedanken aus, ein gattungsgemäßes Instrument mit wenigstens zwei beweglichen Maulteilen, das insbesondere in Art einer an sich bekannten Faß- bzw. Greifzange aufgebaut sein kann, mit einem zusätzlichen Schiebeelement zu versehen, das sich in dem Raum, in dem die Prothese angebracht werden soll, also beispielsweise an der Tracheo-Bronchial-Wand abstützt, während die Zange aus dem Tracheo-Bronchial-System entfernt wird.

Dabei ist es besonders bevorzugt, wenn die Bedienelemente für die Maulteile, die beispielsweise in Art herkömmlicher Handgriffe für medizinische Zange ausgebildet sein können. mit einer Rasteinrichtung versehen sind, die ein Festhalten der Prothese ohne Festhalten bzw. Betätigen der Handgriffe erlaubt. Die Rastung der Handgriffe kann ferner in besonders bevorzugter Weise durch Drücken auf einen Hebel an der Rasteinrichtung aufhebbar sein.

### Kurze Beschreibung der Zeichnung

Die Erfindung wird nachstehend anhand eines Ausführungsbeispiels unter Bezugnahme auf die Zeichnung näher beschrieben, in der zeigen:

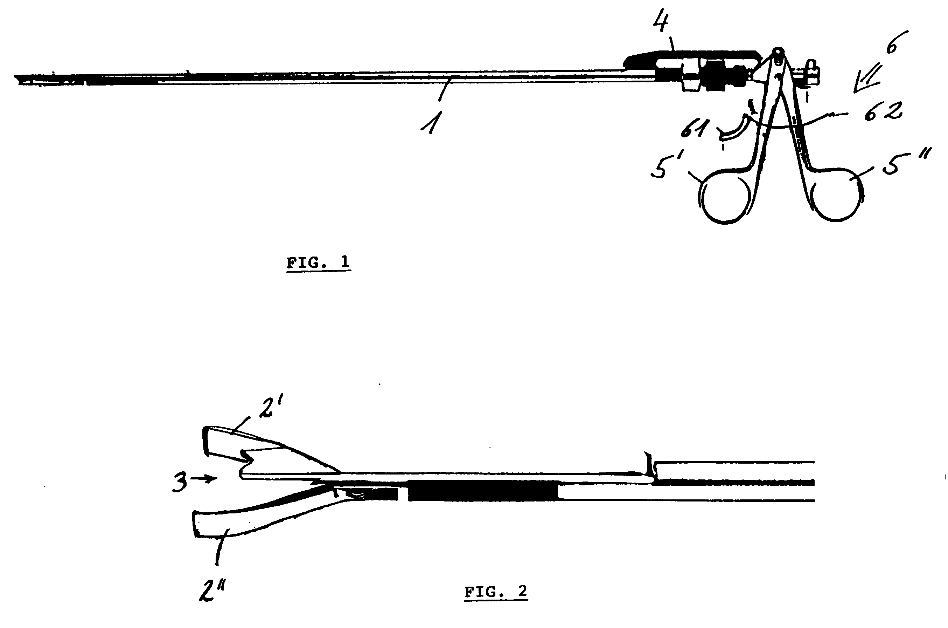

- Fig. 1: eine Seitenansicht eines erfindungsgemäßen Instruments, und
- Fig. 2: vergrößert den distalen Endbereich eines erfindungsgemäßen Instruments.

### Beschreibung eines Ausführungsbeispiels

In den Figuren ist ein erfindungsgemäßes Instrument zur Applikation von Prothesen im Körperinneren und insbesondere von Tracheo-Bronchial-Prothesen dargestellt. Das Instrument weist einen Instrumentenschaft 1 auf, der an seinem distalen Ende zwei bewegliche Maulteile 2' und 2'' trägt. Die Maulteile 2' und 2'' dienen zum Halten einer nichtdargestellten Prothese während des Einbringvorgangs. Ferner ist ein Schiebeelement 3 vorgesehen, das längs des Instrumentenschafts 1 verschiebbar ist, und das zum Halten der Prothese während der Entnahme des Instruments beispielsweise aus dem Tracheo-Bronchial-Raum dient.

Am proximalen Ende des Instrumentenschafts 1 sind die im folgenden näher erläuterten Bedienelemente für die Maulteile 2' und 2'' und das Schiebeelement 3 vorgesehen:

Zum Bewegen der Maulteile sind zwei Handgriffe 5' und 5'' vorgesehen, die in gleicher Weise wie die Handgriffe medizinischer Zangen, beispielsweise von Greif- bzw. Faßzangen ausgebildet sind. Die Handgriffe 5' und 5'' sind mit einer Rasteinrichtung 6 versehen, die ein Festhalten der Prothese ohne Halten der Handgriffe erlaubt. Die Rasteinrichtung weist insbesondere einen Hebel 62 auf, der die beiden Handgriffe 5' und 5'' durchsetzt und durch den die Rastung hergestellt wird. Die Rastung der Handgriffe wird dadurch aufgehoben, daß ein Hebel 61 an der Rasteinrichtung 6 gedrückt wird.

Das Bedienelemente für das Schiebeelement 3 ist eine im proximalen Endbereich des Instrumentenschafts 1 vorgesehene Schiebetaste 4. Ferner ist das Schiebeelement an seinem distalen Ende mit einer V-förmigen Ausnehmung versehen, die eine besonders effiziente Abstützung der Prothese erlaubt.

## Patentansprüche

1. Instrument zur Applikation von Prothesen im Körperinneren und insbesondere von Tracheo-Bronchial-Prothesen, mit
- einem Instrumentenschaft (1),
- wenigstens zwei beweglichen Maulteilen (2',2''), die am distalen Ende des Instrumentenschaftes (1) vorgesehen sind, und die zum Halten der Prothese während des Einbringvorganges dienen, und
- im proximalen Bereich angeordneten Bedienelementen (5',5'',6) für die Maulteile
**gekennzeichnet** durch wenigstens ein Schiebeelement (3), das längs des Instrumentenschaftes (1) verschiebbar ist, und zum Halten der Prothese bei der Entnahme des Instruments dient, und das mittels im proximalen Bereich angeordneter Bedienelemente (4) bedient wird.

2. Instrument nach Anspruch 1,
dadurch **gekennzeichnet**, daß die Bedienelemente für die Maulteile (2' ,2'') Handgriffe (5',5'') für medizinische Zangen sind.

3. Instrument nach Anspruch 2,
dadurch **gekennzeichnet**, daß die Handgriffe (5',5'') mit einer Rasteinrichtung (6) versehen sind, die ein Festhalten der Prothese ohne Halten der Handgriffe erlaubt.

4. Instrument nach Anspruch 3,
dadurch **gekennzeichnet**, daß die Rastung der Handgriffe durch Drücken auf einen Hebel (61) an der Rasteinrichtung (6) aufhebbar ist.

5. Instrument nach Anspruch 3 oder 4,
dadurch **gekennzeichnet**, daß die Rasteinrichtung einen Hebel (62) aufweist, der beide Handgriffe (5',5'') durchsetzt, und der für die Rastung sorgt.

6. Instrument nach einem der Ansprüche 1 bis 5,
dadurch **gekennzeichnet**, daß das Schiebeelement an seinem distalen Ende eine V-förmige Ausnehmung (31) aufweist.

7. Instrument nach einem der Ansprüche 1 bis 6,
dadurch **gekennzeichnet**, daß das Bedienelement für das oder die Schiebeelemente (3) eine im proximalen Endbereich vorgesehene Schiebetaste (4) ist.

## Claims

1. An instrument for the application of prostheses inside the body and, in particular, of tracheobronchial prostheses, having
- an instrument shaft (1),
- at least two moveable jaws (2', 2''), which are provided at the distal end of said instrument shaft (1), and which serve to hold the prosthesis during the insertion procedure,
- operating elements (5',5'', 6) for said jaws disposed in the proximal region,
characterized by at least one sliding element (3), which can be slid along said instrument shaft (1), serving to hold the prosthesis during the removal of said instrument and being operated by the operating elements (4) disposed in the proximal region.

2. An instrument according to claim 1,
**characterized by** said operating elements for said jaws (2', 2'') being handles (5', 5'') of medical tongs.

3. An instrument according to claim 2,
**characterized by** said handles (5', 5'') being provided with a detention device (6), which permits securing the prosthesis without holding the handles.

4. An instrument according to claim 3,
**characterized by** said detention of said handles being releaseable by pressing on a lever (61) on the detention device (6).

5. An instrument according to claim 3 or 4,
**characterized by** said detention device having a lever (62) which passes through both said handles (5', 5'') and provides detention.

6. An instrument according to one of the claims 1 to 5,
**characterized by** said sliding element being provided with a V-shaped recess (31) at the distal end thereof.

7. An instrument according to one of the claims 1 to 6,
**characterized by** said operating element for said sliding element or elements (3) being a sliding button (4) provided in the proximal end region.

## Revendications

1. Instrument pour la mise en place de prothèses à l'intérieur du corps et en particulier de prothèses trachéobronchiques avec
- une tige d'instrument (1),
- au moins deux parties de mâchoire mobiles (2', 2''), qui sont prévues sur l'extrémité distale de la tige de l'instrument (1) et qui servent à maintenir la prothèse pendant l'introduction, et
- des éléments de manoeuvre (5', 5'', 6) disposés dans la zone proximale pour les parties de mâchoire
caractérisé par au moins un élément coulissant (3) pouvant se déplacer le long de la tige (1) de l'instrument et qui sert à maintenir la prothèse lors de l'enlèvement de l'instrument et qui est actionné au moyen d'un élément de manoeuvre (4) disposé dans la zone proximale.

2. Instrument selon la revendication 1,
caractérisé en ce que les instruments de manoeuvre destinés aux parties de mâchoire (2', 2'') sont des poignées (5', 5'') pour pinces médicales.

3. Instrument selon la revendication 2,
caractérisé en ce que les poignées (5', 5'') sont munies d'un dispositif de blocage (6) qui permet de maintenir la prothèse sans tenir les poignées.

4. Instrument selon la revendication 3,
caractérisé en ce que le blocage des poignées peut être supprimé en appuyant sur un levier (61) disposé sur le dispositif de blocage (6).

5. Instrument selon la revendication 3 ou 4,
caractérisé en ce que le dispositif de blocage comporte un levier (62) qui traverse les deux poignées (5', 5'') et qui sert à la mise en blocage.

6. Instrument selon l'une des revendications 1 à 5,
caractérisé en ce que l'élément coulissant comporte sur son extrémité distale un évidement en forme de V.

7. Instrument selon l'une des revendications 1 à 6,
caractérisé en ce que l'élément de manoeuvre pour le ou les éléments coulissant(s) (3) est une touche coulissante (4) prévue dans la zone d'extrémité proximale.
